# FASCICULE DE BREVET EUROPEEN

(11) **EP 2 213 333 B1**
(45) Date de publication et mention de la délivrance du brevet: **04.11.2015**
(21) Numéro de dépôt: 10305073.8
(22) Date de dépôt: 22.01.2010
(51) Int. Cl.: A61Q 5/06, A61K 8/04, A61K 8/31, A61K 8/33, A61K 8/34, A61K 8/81

(54) **Composition de coiffage a effet cire, en aerosol et utilisations**
Zusammensetzung für die Haarpflege mit Wachseffekt, als Spray und entsprechende Anwendungen
Wax-effect hairstyling composition in aerosol form and uses thereof

(30) Priorité: 30.01.2009 FR 0950605
(43) Date de publication de la demande: 04.08.2010
(73) Titulaire: L'Oréal, 75008 Paris (FR)
(72) Inventeur: Gawtrey, Jonathan, 92100, Boulogne (FR); Bebot, Cécile, 92110, Clichy (FR); Rodrigues, Céférino, 93100, Montreuil (FR)
(74) Mandataire: Dossmann, Gérard

(56) Documents cités:
- EP-A- 1 417 952
- JP-A- 2002 370 947
- US-A- 4 223 009
- US-A1- 2004 141 926

## Description

La présente invention concerne une composition cosmétique capillaire, conditionnée dans un dispositif aérosol, comprenant un ou plusieurs polymère(s) fixant(s) anionique(s), un ou plusieurs polyol(s) et un ou plusieurs alcool(s) gras liquide(s).

La présente invention concerne aussi un procédé cosmétique de coiffage comprenant l'application de ladite composition sur les fibres kératiniques, de préférence les fibres kératiniques humaines telles que les cheveux, ainsi que les utilisations de cette composition en coiffage notamment pour conférer aux fibres kératiniques un effet de cire.

Les produits capillaires pour la mise en forme et/ou le maintien des cheveux les plus répandus sur le marché de la cosmétique sont des compositions à pulvériser, telles que les laques et les sprays. Ils sont généralement composés d'un agent propulseur et d'une phase liquide comprenant, dans un milieu cosmétiquement acceptable, au moins un composant fixant dont la fonction est de former des soudures entre les cheveux, en mélange avec divers adjuvants cosmétiques. Généralement, le milieu cosmétiquement acceptable est un milieu alcoolique ou hydro-alcoolique et l'agent propulseur est un gaz liquéfié sous pression réduite ou dissous dans la phase liquide.

Pour des raisons essentiellement écologiques, on cherche à diminuer les teneurs en composés organiques volatils (COV), tels que l'éthanol, présents dans ces compositions. Cependant cette réduction de la quantité de COV ne doit pas s'effectuer au détriment des propriétés de mise en forme et de maintien des cheveux.

Les produits de coiffage à effet cire se présentent majoritairement sous forme de pâtes plus ou moins visqueuses qui s'appliquent sur les cheveux avec les mains. Généralement, ces produits sont conditionnés dans des tubes ou dans des récipients munis d'un couvercle. Ces dispositifs de conditionnement ne sont toutefois pas toujours entièrement satisfaisants : ces dispositifs ne permettent pas toujours de délivrer aisément la composition dans la quantité requise : la composition est souvent appliquée en une quantité trop importante par rapport à la quantité nécessaire ou souhaitée. De plus, il est souvent difficile d'obtenir une répartition homogène de la composition sur l'ensemble de la zone à traiter.

Le problème posé à l'origine de la présente invention est de proposer des compositions cosmétiques capillaires qui procurent un effet de cire aux fibres kératiniques, sans présenter les inconvénients cités ci-dessus.

Des compositions à effet cire conditionnées en aérosol ont déjà été proposées dans les demandes de brevet US 2007/0053847 et FR 2846880.

La demande US 2007/0053847décrit une composition capillaire en aérosol comprenant au moins un polymère fixant anionique, non-ionique, amphotère ou cationique ; de 3 à 25 % en poids d'un éther polyalkylèneglycol d'un glycéride d'acide gras, ou d'un glycéride partiel ; de 1 à 15% en poids d'un alcool gras éthoxylé, saturé ou insaturé, en C₁₂-C₁₈ comprenant de 1 à 3 motifs éthoxy. Cette composition ne permet pas d'apporter un effet cire satisfaisant.

La demande FR 2846880 décrit une composition capillaire en aérosol comprenant de 0,5 à 10 % en poids d'un polymère fixant anionique, au moins 15% en poids d'un polyol de poids moléculaire inférieur à 500, au moins 10% en poids d'un milieu alcoolique ou aqueux, au moins 30% en poids d'un gaz propulseur. Cette composition est délivrée sous la forme d'un spray moussant qui ne permet pas de contrôler la quantité appliquée, ce qui peut induire un effet alourdissant des cheveux.

De manière inattendue et avantageuse, la Demanderesse a mis en évidence la composition cosmétique de préférence anhydre selon l'invention qui permet d'apporter aux fibres kératiniques un effet cire, sans présenter les inconvénients de l'art antérieur.

La composition selon l'invention permet de conférer à la chevelure un effet de coiffant sans alourdissement. Elle permet de construire et de modeler la coiffure toute en apportant du coiffant, du volume et de la discipline. Elle permet aussi un apport de brillance.

La présente invention a ainsi pour objet une composition cosmétique capillaire avec une teneur en eau inférieure ou égale à 10% en poids par rapport au poids total de la composition, conditionnée dans un dispositif aérosol et comprenant :
▪ un ou plusieurs polymères fixants anioniques,
▪ un ou plusieurs polyols,
▪ un ou plusieurs alcools gras liquides,
▪ un ou plusieurs monoalcools inférieurs en C₁-C₄,
▪ un ou plusieurs gaz propulseurs.

La composition selon la présente invention est contenue dans un dispositif aérosol.

Un autre objet de l'invention consiste en un procédé de coiffage des fibres kératiniques, de préférence les fibres kératiniques humaines telles que les cheveux mettant en oeuvre la composition selon l'invention conditionnée dans un dispositif aérosol.

Un troisième objet de l'invention consiste en l'utilisation de la composition cosmétique selon l'invention pour le coiffage, la mise en forme des fibres kératiniques, de préférence les fibres kératiniques humaines et en particulier les cheveux en particulier pour procurer un effet coiffant aux fibres kératiniques.

Les compositions selon la présente invention sont faciles à préparer et à appliquer.

En outre, les compositions selon la présente invention permettent de conférer à la coiffure un maintien naturel et durable.

D'autres caractéristiques, aspects, objets et avantages de l'invention apparaîtront encore plus clairement à la lecture de la description et des exemples qui suivent.

Au sens de la présente demande, on entend par :
« composition à effet de cire », une composition destinée à maintenir et/ou à fixer les fibres kératiniques entre elles par un certain effet gras, sans le durcissement apporté par les sprays de fixation, tout en respectant la brillance naturelle des fibres kératiniques, comme les fibres kératiniques humaines telles que les cheveux ;
« composition cosmétique capillaire », une composition de fixation et/ou de maintien des fibres kératiniques, de préférence les fibres kératiniques humaines telles que les cheveux, des compositions de soin des fibres kératiniques, des compositions de conditionnement des fibres kératiniques telles que des compositions destinées à apporter de la douceur aux fibres kératiniques ou encore des composition de maquillage des fibres kératiniques.

La composition capillaire selon la présente invention peut être utilisée dans une application rincée ou non-rincée. De préférence elle est utilisée dans une application non-rincée.

La composition selon la présente invention dans laquelle sont associés des polymères fixants anioniques, des polyols et des alcools gras liquides permet d'obtenir des aérosols à faible teneur en COV, notamment à faible teneur en alcools inférieurs en C₁-C₄. La teneur en COV de la composition est en effet de préférence inférieure ou égale à 55%, encore plus préférentiellement inférieure ou égale à 50% et de manière encore plus préférée inférieure ou égale à 45% en poids par rapport au poids de la composition.

Tous les polymères fixants anioniques ainsi que leurs mélanges utilisés dans la technique peuvent être utilisés dans les compositions selon la présente demande.

Par « polymère fixant » au sens de la présente demande, on entend tout polymère permettant de conférer une forme à la chevelure ou de maintenir la chevelure dans une forme donnée.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 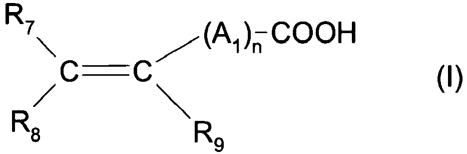 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut aussi citer les terpolymères acide acrylique/acrylate d'éthyle/N-tertiobutylacrylamide vendus notamment sous la dénomination ULTRAHOLD^{®} STRONG par la société BASF. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse ;
E) Les polyacrylamides comportant des groupements carboxylates.

Les homopolymères et copolymères comprenant des groupements sulfoniques comme les polymères comportant des motifs vinylsulfonique, styrène-sulfonique, naphtalène-sulfonique ou acrylamido-alkylsulfonique différents des polyesters sulfoniques ramifiés utilisés selon l'invention.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société Lubrizol.

Les polymères anioniques utilisables selon la présente invention peuvent aussi être des polycondensats comprenant au moins une séquence polyuréthane, ainsi qu'au moins un groupement anionique. Ces polycondensats peuvent ou non comprendre une séquence polysiloxane. Parmi ces polycondensats, on préfère utiliser les polyuréthanes anioniques et en particulier, ceux commercialisés par BASF sous la dénomination Luviset PUR ou Luviset Si PUR.

Les polymères anioniques utilisables selon la présente invention peuvent aussi être des polymères à squelette siliconé et à greffons hydrocarbonés et des polymères à squelette hydrocarboné et à greffons siliconés, ces polymères devant comporter, dans leur structure, au moins un groupement anionique. De préférence, les greffons sont fixés au squelette via un macromonomère comme polymère de ce type, on peut utiliser le polymère VS 30 de la société 3M.

Le(s) polymère(s) fixant(s) anionique(s) utilisé(s) selon l'invention est/sont particulièrement choisi(s) parmi les copolymères d'acide acrylique et méthacrylique ou leurs sels, les copolymères d'acide crotonique, les copolymères d'acides ou d'anhydrides carboxyliques monoinsaturés en C₄-C₈, les polyacrylamaides à groupements carboxylates, les homopolymères ou copolymères à groupements sulfoniques, les polyuréthanes anioniques, et les polymères siliconés greffés anioniques.

Selon l'invention, les polymères fixants anioniques sont de préférence choisis parmi les copolymères d'acide acrylique avec des esters acryliques tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société Lubrizol.

La composition selon la présente invention présente une teneur en polymères fixants anioniques allant de préférence de 0,01% à 7%, plus préférentiellement allant de 0,1% à 6 % et de manière encore préférée allant de 0,5 à 3% en poids, par rapport au poids de la composition.

Les polyols utilisés dans les compositions de l'invention sont de préférence des molécules comportant une chaîne hydrocarbonée en C₃ à C₃₀, interrompue ou non par un ou plusieurs hétéroatomes et au moins substituée par deux groupement hydroxy.

De préférence, la chaîne hydrocarbonée n'est pas interrompue par un hétéroatome, encore plus préférentiellement, le nombre d'atomes de carbone de la chaîne hydrocarbonée est inférieur à 10 et encore plus préférentiellement inférieur à 8.

Préférentiellement, le polyol est un glycol, c'est-à-dire qu'il comporte au moins deux atomes de carbone adjacents, porteur chacun d'au moins un groupement hydroxy.

Parmi les polyols utilisables selon l'invention on peut citer, de préférence, le propylène glycol, le glycérol (glycérine), l'isoprène glycol, le néopentyl glycol, l'hexylène glycol, les polyéthylènes glycol et leurs mélanges.

Le propylène glycol et le glycérol sont particulièrement préférés.

La composition selon l'invention présente une teneur en polyols allant de préférence de 10 à 55 % en poids, de manière préférée allant de 15 à 40 % en poids et de manière encore plus préférée allant de 15 à 35 % en poids par rapport au poids total de la composition.

Par alcool gras au sens de la présente invention on entend un hydrocarbure comportant au moins 8 atomes de carbone et ne comportant à titre de substituant qu'un groupement hydroxy. En particulier ces alcools gras ne comportent pas de groupements oxyalkylénés en particulier oxyéthylénés ou oxypropylénés ou de groupements glycérolés.

Les alcools gras, liquides à une température inférieure à 30°C sont choisis notamment parmi les alcools gras en C₁₀-C₃₀ liquides, linéaires ou ramifiés, saturés ou insaturés. Préférentiellement l'alcool gras liquide est ramifié et/ou insaturé. Par insaturé on entend comportant une ou plusieurs insaturations de préférence éthyléniques. Plus particulièrement, les alcools gras liquides sont choisis parmi l'alcool caprylique, l'alcool n-décylique, l'alcool isostéarylique, l'alcool isocétylique, l'alcool isoarachidylique, l'octyl-2 dodécanol, le butyl-2 octanol, l'alcool oléique, l'alcool linoléylique, l'alcool linolénylique et leurs mélanges. Préférentiellement l'alcool gras liquide est ramifié et/ou insaturé.

De préférence, l'alcool gras est choisi parmi l'alcool isostéarylique, l'alcool isocétylique et l'octyl-2 dodécanol.

La concentration en alcools gras liquides selon l'invention peut aller de préférence de 0,01% à 10%, et de manière préférée de 0,1 à 10% et encore plus préférentiellement de 1 à 5 % en poids par rapport au poids total de la composition.

De préférence le rapport pondéral polyols/alcools gras est supérieur à 1. Encore plus préférentiellement il va de 1,5 à 10.

La composition selon la présente invention est une composition présentant une teneur en eau allant de 0 à 10 % en poids et de préférence allant de 0 à 5 % en poids , mieux de 0 à 3% d'eau par rapport au poids total de la composition. Encore plus préférentiellement la composition de l'invention ne contient pas d'eau ajoutée volontairement. Elle est alors dite anhydre. La seule eau présente alors dans la composition provient alors de l'eau liée apportée par les matières premières hygroscopiques ou par les molécules hydratées introduites dans la composition. Cette eau liée représente alors de préférence moins de 2% du poids total de la composition.

La composition selon la présente invention comprend de préférence au plus 55 %, mieux de 0,01 à 50%, et de manière encore préférée de 0,1 à 45% en poids d' un ou plusieurs monoalcools inférieurs en C₁-C₄ par rapport au poids de la composition.

De préférence, le monoalcool inférieur en C₁-C₄ est choisi parmi l'éthanol, l'isopropanol et leurs mélanges. Encore plus préférentiellement le monoalcool inférieur est l'éthanol.

Le(s) gaz propulseur(s) utilisé(s) dans la composition selon l'invention sont des gaz solubles ou non dans la composition tels que le diméthyl éther, les hydrocarbures fluorés ou non, les gaz liquéfiés usuels ou leurs mélanges. De préférence le gaz utilisé est le diméthyl éther.

La composition selon la présente invention comprend de préférence entre 10 et 50 %, de manière préférée entre 15 et 45 %, et encore plus préférentiellement entre 20 et 40 % en poids d'un ou plusieurs gaz propulseurs par rapport au poids total de la composition.

La composition selon la présente invention peut comprendre en outre un ou plusieurs additifs choisis parmi les silicones ou les dérivés siliconés sous forme soluble, dispersée, micro dispersée ; des agents conditionneurs solubles ou insolubles tels que par exemple les alcools gras solides, les esters gras ; les actifs traitants, les hydratants autres que les polyols utilisés dans les compositions selon la présente invention, les filtres UV, les acides, les bases, les agents plastifiants, les agents solubilisants, les agents conservateurs, les vitamines et les provitamines, les colorants, les pigments, les agents tensio-actifs anioniques, cationiques, non-ioniques, amphotères, les polymères fixants cationiques, non-ioniques, amphotères, les parfums, les agents anticorrosion et leurs mélanges.

L'homme du métier veillera à choisir les éventuels additifs et leur quantité de manière à ce qu'ils ne nuisent pas aux propriétés des compositions de la présente invention

L'invention a également pour objet un procédé de mise en forme des cheveux, comprenant l'application d'une composition cosmétique selon l'invention. En particulier, l'invention concerne un procédé de coiffage comprenant l'application d'une composition selon l'invention sur les cheveux, le rinçage éventuel des cheveux, puis la mise en forme et éventuellement le séchage des cheveux.

De préférence, le procédé de coiffage ou de mise en forme des cheveux comprend l'application d'une composition cosmétique selon l'invention sans rinçage ultérieur puis la mise en forme des cheveux.

L'invention sera mieux comprise à l'aide des exemples conformes à l'invention qui suivent. Ces exemples sont non limitatifs et constituent des modes de réalisation préférentiels des compositions selon l'invention.

### Exemples

Les compositions suivantes ont été réalisées et placées dans un dispositif aérosol :
Les concentrations sont exprimées en gramme de matières actives pour 100 grammes de composition.

**1. Compositions en aérosol conformes à l'invention**

| Composants | Composition 1 | Composition 2 |
|---|---|---|
| Terpolymère acétate de vinyle/tertiobutyle benzoate de vinyle/acide crotonique (¹) | 2 | 2 |
| AMP (²) | 0,2 | 0,2 |
| Propylène glycol | 6 | 6 |
| glycérol | 10 | 10 |
| Octydodécanol (³) | 4 | 4 |
| éthanol | 42,8 | 42,8 |
| Diméthyléther | 35 | 11 |
| 1,1 Difluoroéthane(⁴) | 0 | 24 |

| | | |
|---|---|---|
| (¹) : polymère fixant anionique :Mexomere PW (CHIMEX) (²) 2amino2méthyl 1 propanol (³) _{:} alcool gras liquide (⁴).Dymel 152A (DUPONT) | | |

Les compositions conformes à l'invention sont appliquées sur les cheveux sans rinçage ultérieur. Elles permettent l'obtenir un effet cire satisfaisant, sans alourdissement et de longue durée.

De plus ces compositions ne conduisent pas à une altération des propriétés des cheveux, en particulier les cheveux sont brillants.

**2. Composition en aérosol non conforme à l'invention**

| composants | Composition 3 |
|---|---|
| Terpolymère acétate de vinyle/tertiobutyle benzoate de vinyle/acide crotonique (¹) | 2 |
| AMP (²) | 0,2 |
| Propylène glycol | 6 |
| glycérol | 10 |
| Alcool stéarylique (⁴) | 4 |
| éthanol | 42,8 |
| Diméthyléther | 11 |
| 1,1 Difluoroéthane(⁴) | 24 |

| | |
|---|---|
| (⁴) : alcool gras solide | |

Cette composition non conforme à l'invention, appliquée sans rinçage ultérieure ne permet pas d'obtenir un effet cire convenable tel que celui obtenu avec les compositions conformes à l'invention.

Cette composition conduit à un effet collant sur les mains et assèche la chevelure sans apporter un effet cire.

## Revendications

1. Composition cosmétique capillaire, avec une teneur en eau inférieure ou égale à 10% en poids par rapport au poids total de la composition, conditionnée dans un dispositif aérosol et comprenant :
▪ un ou plusieurs polymères fixants anioniques,
▪ un ou plusieurs polyols,
▪ un ou plusieurs alcools gras liquides,
▪ un ou plusieurs monoalcools inférieurs en C₁-C₄,
▪ un ou plusieurs gaz propulseurs.

2. Composition cosmétique selon la revendication 1 dans laquelle le polymère fixant anionique est choisi parmi les copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupements sulfoniques, les polyuréthanes anioniques, et les polymères siliconés greffés anioniques.

3. Composition cosmétique selon la revendication 1 ou 2 dans laquelle la teneur en polymères fixants anioniques va de 0,01% à 7 % plus préférentiellement de 0,1% à 6 % et de manière préférée de 0,5 à 3% en poids, par rapport au poids de la composition.

4. Composition cosmétique selon l'une quelconque des revendications précédentes dans laquelle le polyol est choisi parmi le propylène glycol, le glycérol, l'isoprène glycol, le néopentyl glycol, l'hexylène glycol, les polyéthylènes glycol et leurs mélanges, de préférence le propylène glycol et le glycérol.

5. Composition cosmétique selon l'une quelconque des revendications précédentes dans laquelle la teneur en polyols va de 10 à 55 % en poids, de préférence de 15 à 40 % en poids et de manière encore plus préférée de 15 à 35 % en poids par rapport au poids total de la composition.

6. Composition cosmétique selon l'une quelconque des revendications précédentes dans laquelle 1(es) alcool(s) gras liquide(s) est/sont choisi(s) parmi l'alcool caprylique, l'alcool n-décylique, l'alcool isostéarylique, l'alcool isocétylique, l'alcool isoarachidylique, l'octyl-2 dodécanol, le butyl-2 octanol, l'alcool oléique,l'alcool linoléylique, l'alcool linolénylique et leurs mélanges.

7. Composition cosmétique selon l'une quelconque des revendications précédentes dans laquelle la concentration en alcools gras liquides va de 0,01% à 10%, et de manière préférée de 0,1% à 10% et encore plus préférentiellement de 1% à 5 % en poids par rapport au poids total de la composition.

8. Composition cosmétique selon l'une quelconque des revendications précédentes dans laquelle le monoalcool inférieur en C₁-C₄ est choisi parmi l'éthanol, l'isopropanol et leurs mélanges et de préférence est l'éthanol.

9. Composition cosmétique selon l'une quelconque des revendications précédentes dans laquelle le ou les monoalcools inférieurs en C₁-C₄ représentent au plus 55% en poids par rapport au poids total de la composition

10. Composition cosmétique selon la revendication précédente comprenant de 0,01% à 50%, et de manière préférée de 0,1% à 45% en poids d'un ou plusieurs alcools inférieurs en C₁-C₄ par rapport au poids total de la composition.

11. Composition cosmétique selon l'une quelconque des revendications précédentes dans laquelle le ou les gaz propulseurs sont choisis parmi le diméthyléther, les hydrocarbures fluorés ou non, les gaz liquéfiés usuels ou leurs mélanges, le gaz propulseur étant de préférence le diméthyléther.

12. Composition cosmétique selon l'une quelconque des revendications précédentes comprenant de préférence entre 10 et 50 %, de manière préférée entre 15 et 45 %, et encore plus préférentiellement entre 20 et 40 % en poids d' un ou plusieurs gaz propulseurs par rapport au poids total de la composition.

13. Composition cosmétique selon l'une quelconque des revendications précédentes dans laquelle le rapport pondéral polyols/alcools gras est supérieur à 1, ce rapport allant de préférence de 1.5 à 10.

14. Utilisation de la composition cosmétique selon l'une quelconque des revendications précédentes pour le coiffage, la mise en forme des fibres kératiniques, en particulier pour procurer un effet coiffant aux fibres kératiniques.

15. Procédé de coiffage comprenant l'application d'une composition selon l'une quelconque des revendications 1 à 13 sur les cheveux, sans rinçage ultérieur puis la mise en forme des cheveux.

## Patentansprüche

1. Haarkosmetikzusammensetzung mit einem Wassergehalt von 10 Gew.-% oder weniger in Bezug auf das Gesamtgewicht der Zusammensetzung, die in einer Aerosolvorrichtung verpackt ist und die Folgendes umfasst:
▪ ein oder mehrere fixierende anionische Polymere,
▪ ein oder mehrere Polyole,
▪ einen oder mehrere flüssige Fettalkohole,
▪ einen oder mehrere niedere C₁-C₄-Monoalkohole,
▪ ein oder mehrere Treibgase.

2. Kosmetikzusammensetzung nach Anspruch 1, wobei das fixierende anionische Polymer aus den Copolymeren der Acryl- und Methacrylsäure oder ihren Salzen, den Copolymeren der Krotonsäure, den Copolymeren von einfach ungesättigten C₄-C₈-Carbonsäuren oder - anhydriden, den Polyacrylamiden mit Carboxylatgruppen, den Homopolymeren oder Copolymeren mit Sulfonsäuregruppen, den anionischen Polyurethanen und den anionischen Pfropfsilikonpolymeren ausgewählt ist.

3. Kosmetikzusammensetzung nach Anspruch 1 oder 2, wobei der Gehalt an fixierenden anionischen Polymeren im Bereich von 0,01 Gew.-% bis 7 Gew.-%, stärker bevorzugt 0,1 Gew.-% bis 6 Gew.-% und bevorzugt 0,5 bis 3 Gew.-% in Bezug auf das Gewicht der Zusammensetzung liegt.

4. Kosmetikzusammensetzung nach einem der vorhergehenden Ansprüche, wobei das Polyol aus Propylenglykol, Glycerin, Isoprenglykol, Neopentylglykol, Hexylenglykol, den Polyethylenglykolen und ihren Mischungen, vorzugsweise aus Propylenglykol und Glycerin, ausgewählt ist.

5. Kosmetikzusammensetzung nach einem der vorhergehenden Ansprüche, wobei der Polyolgehalt im Bereich von 10 bis 55 Gew.-%, vorzugsweise 15 bis 40 Gew.-%, noch stärker bevorzugt 15 bis 35 Gew.-% in Bezug auf das Gesamtgewicht der Zusammensetzung liegt.

6. Kosmetikzusammensetzung nach einem der vorhergehenden Ansprüche, wobei der flüssige Fettalkohol bzw. die flüssigen Fettalkohole aus Caprylalkohol, n-Decylalkohol, Isostearylalkohol, Isocetylalkohol, Isoarachidylalkohol, 2-Octyldodecanol, 2-Butyloctanol, Oleylalkohol, Linoleylalkohol Linolenylalkohol und ihren Mischungen ausgewählt ist/sind.

7. Kosmetikzusammensetzung nach einem der vorhergehenden Ansprüche, wobei die Konzentration an flüssigen Fettalkoholen im Bereich von 0,01 Gew.-% bis 10 Ges.-%, bevorzugt 0,1 Gew.-% bis 10 Gew.-% und noch stärker bevorzugt 1 Gew.-% bis 5 Gew.-% in Bezug auf das Gesamtgewicht der Zusammensetzung liegt.

8. Kosmetikzusammensetzung nach einem der vorhergehenden Ansprüche, wobei der niedere C₁-C₄-Monoalkohol aus Ethanol, Isopropanol und ihren Mischungen ausgewählt ist und vorzugsweise Ethanol ist.

9. Kosmetikzusammensetzung nach einem der vorhergehenden Ansprüche, wobei der/die niedere(n) C₁-C₄-Monoalkohol(e) maximal 55 Gew.-% in Bezug auf das Gesamtgewicht der Zusammensetzung ausmacht/ausmachen.

10. Kosmetikzusammensetzung nach dem vorhergehenden Anspruch, die 0,01 Gew.-% bis 50 Gew,-%, bevorzugt 0,1 Gew.-% bis 45 Gew.-%, an einem oder mehreren niederen C₁-C₄-Alkoholen in Bezug auf das Gesamtgewicht der Zusammensetzung umfasst.

11. Kosmetikzusammensetzung nach einem der vorhergehenden Ansprüche, wobei das Treibgas bzw. die Treibgase aus Dimethylether, gegebenenfalls fluorierten Kohlenwasserstoffen, den üblichen verflüssigten Gasen oder ihren Mischungen ausgewählt ist oder sind, wobei das Treibgas vorzugsweise Dimethylether ist.

12. Kosmetikzusammensetzung nach einem der vorhergehenden Ansprüche, die vorzugsweise zwischen 10 und 50 Gew.-%, bevorzugt zwischen 15 und 45 Gew.-% und noch stärker bevorzugt zwischen 20 und 40 Gew.-% an einem oder mehreren Treibgasen in Bezug auf das Gesamtgewicht der Zusammensetzung umfasst.

13. Kosmetikzusammensetzung nach einem der vorhergehenden Ansprüche, wobei das Gewichtsverhältnis Polyole/Fettalkohole größer als 1 ist, wobei dieses Verhältnis vorzugsweise im Bereich von 1,5 bis 10 liegt.

14. Verwendung der Kosmetikzusammensetzung nach einem der vorhergehenden Ansprüche für das Styling, das Formen von Keratinfasern, insbesondere zum Hervorrufen einer Styling-Wirkung auf Keratinfasern.

15. Stylingverfahren, bei dem eine Zusammensetzung nach einem der Ansprüche 1 bis 13 ohne späteres Spülen auf das Haar appliziert wird und das Haar anschließend in Form gebracht wird.

## Claims

1. Hair cosmetic composition with a water content of less than or equal to 10% by weight, with respect to the total weight of the composition, packaged in an aerosol device and comprising:
▪ one or more anionic fixing polymers,
▪ one or more polyols,
▪ one or more liquid fatty alcohols,
▪ one or more lower C1-C4 monoalcohols,
▪ one or more propellant gases.

2. Cosmetic composition according to Claim 1, in which the anionic fixing polymer is chosen from copolymers of acrylic or methacrylic acid or their salts, copolymers of crotonic acid, copolymers of C4-C8 monounsaturated carboxylic acids or anhydrides, polyacrylamides comprising carboxylate groups, homopolymers or copolymers comprising sulpho groups, anionic polyurethanes and anionic grafted silicone polymers.

3. Cosmetic composition according to Claim 1 or 2, in which the content of anionic fixing polymers ranges from 0.01% to 7% by weight, more preferentially from 0.1 to 6% by weight and preferably from 0.5 to 3% by weight, with respect to the weight of the composition.

4. Cosmetic composition according to any one of the preceding claims, in which the polyol is chosen from propylene glycol, glycerol, isoprene glycol, neopentyl glycol, hexylene glycol, polyethylene glycols and their mixtures, preferably propylene glycol and glycerol.

5. Cosmetic composition according to any one of the preceding claims, in which the content of polyols ranges from 10 to 55% by weight, preferably from 15 to 40% by weight and more preferably still from 15 to 35% by weight, with respect to the total weight of the composition.

6. Cosmetic composition according to any one of the preceding claims, in which the liquid fatty alcohol(s) is/are chosen from caprylic alcohol, n-decyl alcohol, isostearyl alcohol, isocetyl alcohol, isoarachidyl alcohol, 2-octyldodecanal, 2-butyloctanol, oleyl alcohol, linoleyl alcohol, linolenyl alcohol and their mixtures.

7. Cosmetic composition according to any one of the preceding claims, in which the concentration of liquid fatty alcohols ranges from 0.01 to 10% by weight, preferably from 0.1 to 10% by weight and more preferably still from 1 to 5% by weight, with respect to the total weight of the composition.

8. Cosmetic composition according to any one of the preceding claims, in which the lower C1-C4 monoalcohol is chosen from ethanol, isopropanol and their mixtures and is preferably ethanol.

9. Cosmetic composition according to any one of the preceding claims, in which the lower C1-C4 monoalcohol or monoalcohols represent at most 55% by weight, with respect to the total weight of the composition.

10. Cosmetic composition according to the preceding claim, comprising from 0.01 to 50% by weight and preferably from 0.1 to 45% by weight of one or more lower C1-C4 alcohols, with respect to the total weight of the composition.

11. Cosmetic composition according to any one of the preceding claims, in which the propellant gas or gases are chosen from dimethyl ether, fluorinated or nonfluorinated hydrocarbons, standard liquefied gases or their mixtures, the propellant gas preferably being dimethyl ether.

12. Cosmetic composition according to any one of the preceding claims, preferably comprising between 10 and 50% by weight, preferably between 15 and 45% by weight and more preferably still between 20 and 40% by weight of one or more propellant gases, with respect to the total weight of the composition.

13. Cosmetic composition according to any one of the preceding claims, in which the polyols/fatty alcohols ratio by weight is greater than 1, this ratio preferably ranging from 1.5 to 10.

14. Use of the cosmetic composition according to any one of the preceding claims in the styling or shaping of keratinous fibres, in particular for providing keratinous fibres with a styling effect.

15. Styling method, comprising the application of a composition according to any one of Claims 1 to 13 to the hair, without subsequent rinsing, and then the shaping of the hair.
